# EUROPÄISCHE PATENTSCHRIFT

(11) **EP 4 110 430 B1**
(45) Veröffentlichungstag und Bekanntmachung des Hinweises auf die Patenterteilung: **03.04.2024**
(21) Anmeldenummer: 22708883.8
(22) Anmeldetag: 22.02.2022
(51) Int. Cl.: A61M 5/145, A61M 5/14

(54) **VORRICHTUNG ZUR VERABREICHUNG VON MEDIZINISCHER FLÜSSIGKEIT MIT KÜHLKÖRPER MIT KÜHLRIPPEN**
APPARATUS FOR THE ADMINISTRATION OF MEDICAL LIQUIDS WITH A COOLING BODY COMPRISING COOLING FINS
DISPOSTIF D'ADMINISTRATION DES MEDICAMENTS AVEC UN CORPS DE REFROIDISSEMENT COMPRENANT DES AILETTES DE REFROIDISSEMENT

(30) Priorität: 24.02.2021 DE 102021104413
(43) Veröffentlichungstag der Anmeldung: 04.01.2023
(73) Patentinhaber: B. Braun Melsungen AG, 34212 Melsungen (DE)
(72) Erfinder: ERLEN, Christoph, 34132 Kassel (DE); SCHÖFFEL, Gerhard, 89134 Blaustein (DE); SCHÜTZ, Joachim, 36041 Fulda (DE); WOLF, Daniel, 89346 Bibertal (DE)
(74) Vertreter: Winter, Brandl - Partnerschaft mbB
(86) Internationale Anmeldenummer: PCT/EP2022/054345
(87) Internationale Veröffentlichungsnummer: WO 2022/180013

(56) Entgegenhaltungen:
- EP-A1- 3 696 908
- US-A- 4 964 785
- US-A1- 2007 233 003
- US-A1- 2013 281 965
- US-B2- 10 034 975

## Beschreibung

### Technisches Gebiet

Die vorliegende Offenbarung betrifft eine Vorrichtung zur Verabreichung von medizinischer Flüssigkeit, insbesondere eine Infusionspumpe. Ferner betrifft die vorliegende Offenbarung ein System aus einer Haltevorrichtung, wie einem Stativ, und einer solchen Vorrichtung.

Infusionspumpen werden im Wesentlichen zwischen Spritzenpumpen und Schlauchpumpen unterschieden. Bei Spritzenpumpen wird eine mit der zu verabreichenden Flüssigkeit gefüllte Spritze durch einen Antrieb bewegt, so dass ein Spritzenkolben der Spritze mit gesteuerter Vorschubgeschwindigkeit bewegt wird, um die Flüssigkeit aus der Spritze heraus zu fördern. Bei Schlauchpumpen wird ein mit der zu verabreichenden Flüssigkeit gefüllter Schlauch durch einen Antrieb, etwa mittels einer Peristaltik, bewegt, um die Flüssigkeit aus dem Schlauch heraus zu fördern.

Solche Infusionspumpen weisen eine Vielzahl von (Elektronik-)Komponenten auf, deren Betrieb Wärme erzeugt. Eine solche Infusionspumpe ist beispielsweise aus der US 10,034,975 B2 bekannt, die eine Infusionspumpe mit einem Gehäuse und einer darin angeordneten, Wärme erzeugenden Komponente aufweist.

Weiterhin offenbart US 4 964 785 A eine Pumpenanordnung allgemeiner Art mit einem Pumpengehäuse und einem wärmeerzeugenden Element. Das Pumpengehäuse und ein Motorgehäuse sind fest miteinander gekoppelt. Am Motorgehäuse ist ein passiver Kühlkörper in Form von Rippen vorgesehen, die sich entlang der Axialrichtung des Motorgehäuses erstrecken.

US 2007/233003 A1 offenbart eine Spülpumpe mit einem Gehäuse und mit einer wärmeerzeugenden Komponente als Teil einer Energiequelle. Eine an der Rückseite der Pumpe vorgesehene C-förmige Klemme kann aus einem wärmeleitenden Metall gefertigt sein und somit als Kühlkörper (ohne Kühlrippen) dienen.

US 2013/281965 A1 offenbart eine Spritzenpumpe mit einer Energiequelle, die Wärme erzeugt, und einem Pumpengehäuse, das selbst als Kühlkörper verwendet wird. An dem Pumpengehäuse ist keine zusätzliche passive Kühlstruktur vorgesehen.

US 10 034 975 82 B2 offenbart eine Infusionspumpe mit einem Gehäuse und einer darin angeordneten, Wärme erzeugenden Komponente, wobei auf der Gehäuseaußenseite ein mit der Komponente thermisch verbundener, passiver Kühlkörper ohne Kühlrippen vorhanden ist.

EP 3 696 908 A1 offenbart eine Montagevorrichtung (Koppelabschnitt) zur Montage eines Elektronikmoduls (Vorrichtung, die eine Wärme erzeugende Komponente aufweist) an einer Struktur (Gegenkoppelabschnitt). Das Elektronikmodul weist einen Kühlkörper mit Kühlrippen auf. Die Montagevorrichtung befindet sich seitlich des Kühlkörpers. Die Montagevorrichtung ist nicht zur wärmeleitenden Kopplung mit der Struktur vorgesehen.

Die erzeugte Wärme muss an die Umgebung abgeführt werden, um die Temperatur der Infusionspumpe innerhalb eines für alle Komponenten zulässigen Bereichs zu halten und die Komponenten vor Überhitzung zu schützen. Dazu ist in bekannten Infusionspumpen ein in dem Infusionspumpengehäuse angeordneter Lüfter/Ventilator eingebaut, um die Wärme mittels Luftkühlung abzutransportieren, indem entlang der Oberflächen der wärmeerzeugenden (Elektronik-)Komponenten strömende Luft aus dem Infusionspumpengehäuse herausgeblasen wird. Nachteilig daran ist jedoch, dass zum Herausführen der Luft aus dem Infusionspumpengehäuse Öffnungen in diesem vorgesehen sein müssen. Durch diese Öffnungen können wiederum Flüssigkeiten oder andere unerwünschte äußere Einflüsse in das Infusionspumpengehäuse eindringen, was zur Gewährleistung der Funktionalität der Infusionspumpe zu vermeiden ist. Ein weiterer Nachteil besteht darin, dass der Lüfter sowie ein gegebenenfalls zur Kühlung der Luft vorhandener Kälteerzeuger mit elektrischer Energie versorgt werden müssen, so dass es bei fehlender Energieversorgung zu einem Ausfall der Kühlung kommen kann. Zudem benötigen der Lüfter und der Kälteerzeuger zusätzlichen Bauraum innerhalb des Infusionspumpengehäuses. Aufgrund der kompakten Bauform der Infusionspumpe und ihrer begrenzten Akkukapazität ist eine solche Luftkühlung der (Elektronik-) Komponenten demnach nicht praktikabel.

Es ist also Aufgabe der vorliegenden Offenbarung, eine Vorrichtung zur Verabreichung von medizinischer Flüssigkeit, insbesondere eine Infusionspumpe, bereitzustellen, bei der jederzeit gewährleistet werden kann, dass die Temperatur innerhalb der Vorrichtung eine zulässige Maximaltemperatur aller in der Vorrichtung enthaltener Komponenten nicht übersteigt. Gleichzeitig soll die Vorrichtung einen kompakten Aufbau besitzen, einfach zu reinigen und/oder sterilisieren sein sowie zur Sicherstellung der Funktionalität die innerhalb eines Vorrichtungsgehäuses angeordneten Komponenten vor äußeren Einflüssen schützen. Auch ist es die Aufgabe der vorliegenden Offenbarung, ein System mit einer solchen Vorrichtung sowie einer Haltervorrichtung zum Halten der Vorrichtung bereitzustellen.

Die Aufgabe der vorliegenden Offenbarung wird durch eine Vorrichtung mit den Merkmalen des unabhängigen Patentanspruchs 1 sowie durch ein System mit den Merkmalen des nebengeordneten Systemanspruchs gelöst. Vorteilhafte Weiterbildungen sind Gegenstand der Unteransprüche.

Insbesondere ist die Vorrichtung zur Verabreichung von medizinischer Flüssigkeit als eine Infusionspumpe ausgebildet. Beispielsweise kann die Vorrichtung als eine Spritzenpumpe oder als eine Schlauchpumpe ausgebildet sein.

Die Vorrichtung weist ein Gehäuse sowie zumindest eine, insbesondere bei Gebrauch der Vorrichtung, Wärme erzeugende Komponente, d.h. eine zu kühlende Komponente, auf. Die Komponente ist innerhalb des Gehäuses angeordnet. Beispielsweise kann die Komponente als ein Elektronikbauteil ausgebildet sein. Auch kann die Vorrichtung mehrerer solcher zumindest bei Gebrauch der Vorrichtung Wärme erzeugenden Komponenten aufweisen. Zu den Wärme erzeugenden Komponenten können in einer Infusionspumpe beispielsweise ein Netzteil zur Stromversorgung anderer elektrisch betriebener Bauteile und/oder ein Akkumulator zur Stromversorgung anderer elektrisch betriebener Bauteile gezählt werden. Zudem kann ein Funkmodul, wie ein WLAN-Modul oder ein Bluetooth-Modul, zur Signalübertragung eine Wärme erzeugende Komponente sein. Auch kann ein elektromotorisches Bauteil, insbesondere ein Antrieb zur Förderung der Flüssigkeit, eine Wärme erzeugende Komponente sein. Zu den Wärme erzeugenden Komponenten, insbesondere einer Infusionspumpe, können beispielsweise auch eine Anzeigeeinheit zur Anzeige betriebsrelevanter Daten, ein Steuergerät zum Steuern der Vorrichtung, beispielsweise der Anzeigeeinheit oder des Antriebs, oder dergleichen gezählt werden. Mit anderen Worten wird in dem Inneren des Gehäuses der Vorrichtung Wärme erzeugt, die aus dem Gehäuse abgeführt werden muss.

Gemäß der vorliegenden Offenbarung weist die Vorrichtung einen auf einer Außenseite des Gehäuses angebrachten, passiven Kühlkörper auf. Unter einem passiven Kühlkörper ist ein Bauteil zu verstehen, das mittels passiver Kühlung Wärme an die Umgebung abgibt. Durch die Anbringung des Kühlkörpers auf der Außenseite des Gehäuses kann der Kühlkörper die Wärme direkt an die Umgebung abgeben. Dabei ist passive Kühlung die Kühlung von Maschinen, elektrischen und elektronischen Geräten und Bauteilen allein auf der Basis von freier/natürlicher Konvektion und Wärmestrahlung, bei der die Abwärme an die umgebende Luft abgegeben bzw. abgestrahlt wird. Im Gegensatz dazu wird bei einer aktiven Kühlung die Wärmeenergie von der zu kühlenden Komponente mit Hilfe eines Lüfters oder einer Pumpe abtransportiert. Der Vorteil der passiven Kühlung gegenüber der aktiven Kühlung liegt vorrangig darin, dass keine elektrische Energie zur Kühlung erforderlich ist.

Gemäß der vorliegenden Offenbarung ist der Kühlkörper thermisch, insbesondere wärmeleitend, mit der Komponente verbunden. Das heißt also, dass die Wärme durch Wärmeleitung aus dem Inneren des Gehäuses zu dem Kühlkörper auf der Außenseite transportiert wird, um dort an die Umgebung abgegeben zu werden. Mit anderen Worten wird zur Wärmeübertragung innerhalb des Gehäuses eine andere Art eines Wärmetransportvorgangs, nämlich die Wärmeleitung durch mechanische Berührung, als zur Wärmeübertragung außerhalb des Gehäuses, nämlich Konvektion und/oder Wärmestrahlung, gezielt eingesetzt. Somit wird im Gegensatz zu dem Stand der Technik nicht mehr eine durch einen Lüfter erzwungene Konvektion innerhalb des Gehäuses genutzt, um die Wärme durch Mitführen in der nach außen beförderten Luft abzugeben. Dies hat den Vorteil, dass in dem Gehäuse keine Öffnungen mehr vorgesehen werden müssen, um die Luft herausbefördern zu können, da die Wärme durch direkte mechanische Berührung zu der Außenseite der Vorrichtung übertragen wird. Dadurch können auch keine unerwünschten Medien durch die Öffnungen in das Gehäuse eintreten, so dass die Reinigbarkeit und Sterilisierbarkeit der Vorrichtung erheblich gesteigert werden kann. Ein weiterer entscheidender Vorteil liegt darin, dass keine elektrische Energie genutzt werden muss, um einen solchen Lüfter anzutreiben, so dass die Kühlung der Komponente/n unabhängig von der Stromversorgung bzw. Akkukapazität und somit ausfallsicher ist.

Gemäß der vorliegenden Offenbarung weist der Kühlkörper Kühlrippen auf. Durch das Vorsehen der Kühlrippen kann die Oberfläche des Kühlkörpers vergrößert werden und somit die Wärmeübertragung an die Kühlung verbessert werden.

Gemäß einer bevorzugten Ausführungsform kann die Vorrichtung ein innerhalb des Gehäuses angeordnetes Wärmeleitprofil, wie ein Wärmeleitblech oder dergleichen, aufweisen. Das Wärmeleitprofil kann, vorzugsweise direkt, d.h. mit mechanischem Kontakt, mit der Wärme erzeugenden/zu kühlenden Komponente thermisch verbunden sein. Das Wärmeleitprofil kann, vorzugsweise direkt, d.h. mit mechanischem Kontakt, mit dem Kühlkörper thermisch verbunden sein. Durch die wärmeleitende, vorzugsweise direkte, Verbindung durch das Wärmeleitprofil wird die Wärme der Komponente auf geeignete und einfache Weise nach außen transportiert. Insbesondere kann das Wärmeleitprofil flächig anliegend mit der Komponente und/oder dem Kühlkörper verbunden sein. Durch den flächigen Kontakt und die dadurch entstehende Kontaktfläche kann die übertragbare Wärmeleistung erhöht werden. Vorzugsweise kann das Wärmeleitprofil aus einem Material ausgebildet sein, dessen Wärmeleitfähigkeit größer als 10 W/mK ist, bevorzugt größer als 50 W/mK ist, weiter bevorzugt größer als 100 W/mK ist. Insbesondere kann das Wärmeleitprofil aus einem Metall ausgebildet sein, beispielsweise aus Aluminium oder Kupfer, alternativ aus einem keramischen Werkstoff, insbesondere aus einer technischen Keramik, wie Aluminiumoxid oder Aluminiumnitrid.

Gemäß einer Weiterbildung der bevorzugten Ausführungsform kann der Kühlkörper eine vorzugsweise plane Kontaktfläche aufweisen, die an einer vorzugsweise planen Kontaktfläche des Wärmeleitprofils anliegt oder über eine thermische Schicht, wie einer Wärmeleitpaste, mit einer vorzugsweise planen Kontaktfläche des Wärmeleitprofils verbunden ist. Dabei kann der Kühlkörper beispielsweise eine Aussparung in dem Gehäuse durchgreifend angeordnet sein. Dies hat den Vorteil, dass ein thermischer Widerstand des Gehäusematerials umgangen wird.

Gemäß einer Weiterbildung der bevorzugten Ausführungsform kann das Wärmeleitprofil mehrere Wärme erzeugenden Komponenten mit dem Kühlkörper thermisch verbinden. Dies hat den Vorteil, dass die Anzahl an Bauteilen so gering wie möglich gehalten wird, wenn ein Wärmeleitprofil für den Wärmeabtransport mehrerer Komponenten fungieren kann. Vorzugsweise können die mehreren Komponenten benachbart angeordnet sein. Dadurch kann das Wärmeleitprofil so kurz wie möglich ausgebildet werden, so dass die Wärme nur über eine geringere Strecke abtransportiert werden muss.

Gemäß einer Weiterbildung der bevorzugten Ausführungsform kann das Wärmeleitprofil beabstandet zu dem Gehäuse angeordnet sein. Das heißt, dass das Wärmeleitprofil vorzugsweise ausschließlich den Kühlkörper und die Komponente/n kontaktiert. Dadurch kann sichergestellt werden, dass die Wärme nicht über das Gehäuse übertragen wird, sondern gezielt entlang des Wärmeleitprofils, vorzugsweise auf kürzestem Weg, zu dem Kühlkörper übertragen wird.

Gemäß der vorliegenden Offenbarung weist der Kühlkörper einen Koppelabschnitt, der als Teil des Kühlkörpers ausgebildet ist, zur wärmeleitenden Kopplung mit einem externen Gegenkoppelabschnitt auf. Das heißt, dass der Kühlkörper vorgesehen und ausgebildet ist, zusätzlich zu der Wärmeabgabe über freie/natürliche Konvektion und Wärmestrahlung, die Wärme durch Wärmeleitung an den Gegenkoppelabschnitt, der vorzugsweise als ein Wärmeabtransportbauteil fungiert, zu übertragen. Somit werden alle Arten von Wärmetransportvorgängen genutzt, um die Wärme in besonders effizienter, aber passiver Weise abzuführen.

Gemäß der vorliegenden Offenbarung hat der Koppelabschnitt eine, vorzugsweise plane, Kontaktfläche, die vorgesehen und ausgebildet ist, um eine Oberfläche des Gegenkoppelabschnitts, vorzugsweise direkt, insbesondere flächig anliegend, wärmeleitend zu kontaktieren. Durch den flächigen Kontakt und die dadurch entstehende Kontaktfläche kann die übertragbare Wärmeleistung erhöht werden.

Gemäß der Erfindung sind die Kühlrippen in Gebrauchslage der Vorrichtung vertikal ausgerichtet.

Durch die vertikale Ausrichtung wird die schwerkraftbedingte natürliche/freie Konvektion begünstigt.

Mit anderen Worten kann der Kühlkörper sowohl eine bereichsweise zur Wärmekonvektion und/oder Wärmestrahlung optimierte Oberflächenstruktur, in Form der Kühlrippen, als auch eine bereichsweise zur Wärmeleitung optimierte Oberflächenstruktur, in Form der planen Kontaktfläche, aufweisen.

Gemäß der vorliegenden Offenbarung ist der Koppelabschnitt zwischen den Kühlrippen angeordnet. Dies hat den Vorteil, dass die durch die Kühlrippen bedingte natürliche Konvektion genutzt werden kann, um den Koppelabschnitt und den darin aufnehmbaren oder aufgenommenen Gegenkoppelabschnitt zu umströmen, so dass auch der Koppelabschnitt bzw. der Gegenkoppelabschnitt zum Wärmeabtransport durch Konvektion beiträgt. Somit kann ein besonders effizientes System bereitgestellt werden.

Gemäß einer bevorzugten Ausführungsform kann die Vorrichtung einen Befestigungsabschnitt zur Anbringung der Vorrichtung an einer externen Haltevorrichtung, wie an einer Stativklemme eines Stativs, aufweisen. Dies hat den Vorteil, dass die Vorrichtung im Raum befestigt und/oder mit der Haltevorrichtung im Raum bewegt werden kann. Gemäß einer Weiterbildung der bevorzugten Ausführungsform kann der Befestigungsabschnitt durch den Koppelabschnitt des Kühlkörpers gebildet sein, und der externe Gegenkoppelabschnitt durch die Haltevorrichtung gebildet sein. Das heißt, dass der Koppelabschnitt gleichzeitig zur Befestigung der Vorrichtung dient. Dies hat den Vorteil, dass der Koppelabschnitt zur Befestigung in Kontakt mit der Haltevorrichtung stehen muss, so dass die Funktion des Wärmeabtransports integral gelöst wird. Ein weiterer Vorteil liegt darin, dass der Koppelabschnitt im befestigten Zustand durch die Haltevorrichtung abgedeckt ist und somit nicht unabsichtlich mit der Hand kontaktiert werden kann. Da eine Befestigung der Vorrichtung üblicherweise über eine metallische Stativklemme oder direkt über ein metallisches Stativ erfolgt, ist die wärmeleitende Kontaktierung durch die Haltervorrichtung mit guten Wärmeleitungseigenschaften ohne Änderung an dem vorliegenden System möglich.

Gemäß einer Weiterbildung der bevorzugten Ausführungsform kann der Befestigungsabschnitt eine Schiene aufweisen, die vorgesehen und ausgebildet ist, um die Vorrichtung, insbesondere werkzeuglos, formschlüssig an der Haltevorrichtung zu befestigen. So kann eine einfache Befestigung sichergestellt werden. Insbesondere kann eine durch die Schiene geführte Einsteckrichtung des Befestigungsabschnitts an der Haltevorrichtung vorzugsweise einer Ausrichtung der Kühlrippen und/oder einer Vertikalrichtung entsprechen. Dies hat den Vorteil, dass die Befestigung der Vorrichtung der freien Konvektion nicht entgegenwirkt, etwa indem sie eine Luftumströmung des Befestigungsabschnitts verhindert.

Gemäß einer Weiterbildung der bevorzugten Ausführungsform kann der Kühlkörper, insbesondere der Koppelabschnitt, an einer (in Gebrauchslage der Vorrichtung gesehenen) Rückseite des Gehäuses angeordnet sein. Somit wird das Risiko eines händischen Kontaktierens des (heißen) Koppelabschnitts verringert. Alternativ oder zusätzlich kann der Kühlkörper einen den Koppelabschnitt abdeckenden und nach außen isolierenden Berührungsschutz aufweist. So wird ein Verbrennen durch unbeabsichtigtes Kontaktieren verhindert. Der Berührungsschutz kann einen Einschiebeschlitz für die Haltevorrichtung aufweisen, so dass die Befestigung an der Haltevorrichtung sichergestellt bleibt.

Gemäß einer bevorzugten Ausführungsform kann das Gehäuse im Wesentlichen flüssigkeitsdicht sein. Dies hat den Vorteil, dass das Eindringen von unerwünschten Medien verhindert werden kann. Vorzugsweise kann das Gehäuse vorzugsweise Haltevorrichtung aus einem Metall ausgebildet sein, insbesondere aus Aluminium oder Kupfer. Alternativ kann die Haltevorrichtung aus einem keramischen Werkstoff, insbesondere aus einer technischen Keramik, wie Aluminiumoxid oder Aluminiumnitrid, ausgebildet sein. Dadurch weist die Haltevorrichtung gute Wärmeleitungseigenschaften auf.

Mit anderen Worten betrifft die vorliegende Offenbarung eine Vorrichtung zur Verabreichung von medizinischer Flüssigkeit, insbesondere eine Infusionspumpe, wie eine Spritzenpumpe oder eine Schlauchpumpe, mit einer lüfterlosen, passiven Kühlung der Geräteelektronik ((Elektronik-)Komponenten). Dabei wird die von den Elektronikbauteilen ((Elektronik-)Komponenten) erzeugte Wärme mit Hilfe von Wärmeleitprofilen an einen (außenseitig an einem Gehäuse der Vorrichtung angebrachten) passiven Kühlkörper abgeführt. Der Kühlkörper kann vorzugsweise an einer Rückseite der Vorrichtung angebracht sein. Weiterhin kann der Kühlkörper eine Adaption (Halterfunktion) für eine Stativklemme aufweisen. Das heißt, dass der Kühlkörper gleichzeitig einen Adapter aufweisen/ausbilden kann, der dazu eingerichtet ist, die Vorrichtung an einem Stativ zu halten und der weiter optimiert ist, auf diesem Wege mittels Wärmeleitung Wärme auf das Stativ zu übertragen und somit die Kühlleistung zu erhöhen. Zudem kann eine Oberflächenstruktur des Kühlkörpers für den Kühlprozess/zur Wärmekonvektion optimiert sein. Solche Kühlkörper sind an sich schon aus anderen technischen Gebieten bekannt und werden beispielsweise in Computern und Verbrennungsmaschinen zur Abfuhr von Wärme aus elektrischer Energie an die Umgebung eingesetzt. In der Medizintechnik, insbesondere im Bereich von Infusionspumpen, ist die Verwendung von Kühlkörpern jedoch bisher nicht bekannt. Zusätzlich können die wärmeerzeugenden Elektronikkomponenten der Vorrichtung vorzugsweise nah beieinanderliegend angeordnet und thermisch an ein Wärmeleitprofil/-blech angebunden sein. Das Wärmeleitprofil/-blech ist thermisch mit dem außenliegenden Kühlkörper verbunden, so dass die Wärme mittels natürlicher Konvektion und Wärmestrahlung an die Umgebung abgegeben wird. So kann die Temperatur in dem für alle Bauteile zulässigen Bereich gehalten werden und gleichzeitig eine gute Reinigbarkeit aller für Flüssigkeiten zugänglichen Bereiche sichergestellt werden. Darüber hinaus kann die Kühlung somit ausfallsicher und ohne Verbrauch von elektrischer Energie funktionieren.

### Kurzbeschreibung der Figuren

Fig. 1 ist perspektivische Darstellung einer Vorrichtung zum Verabreichen von medizinischen Flüssigkeiten gemäß der vorliegenden Offenbarung,
Fig. 2 ist eine perspektivische Schnittdarstellung der Vorrichtung, und
Fig. 3 ist eine schematische Darstellung eines Systems aus einer Haltevorrichtung und der Vorrichtung.

### Beschreibung einer bevorzugten Ausführungsform

Nachstehend wird eine bevorzugte Ausführungsform der vorliegenden Offenbarung auf der Basis der zugehörigen Figuren beschrieben.

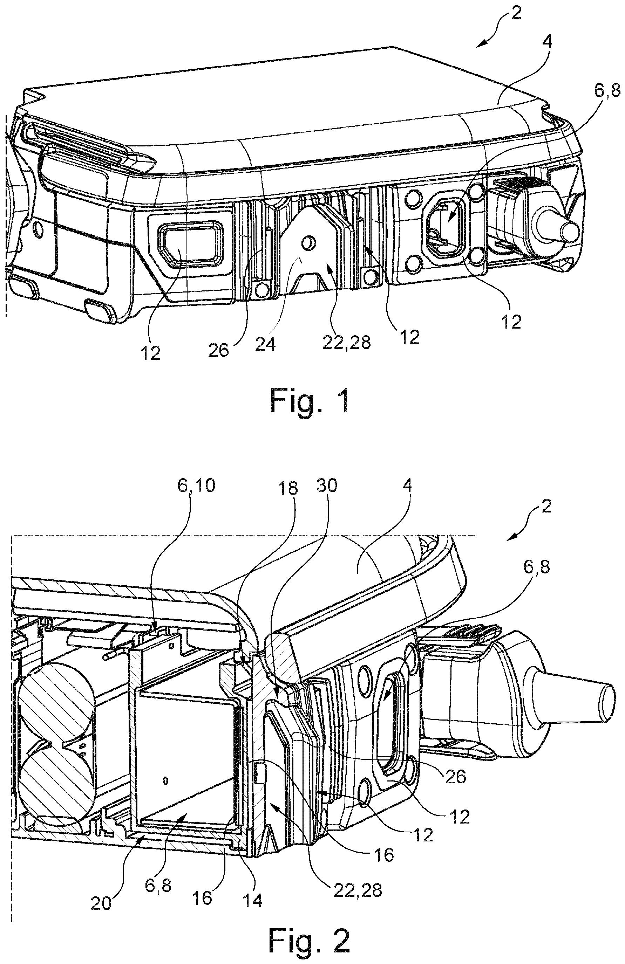

Fign. 1 und 2 zeigen eine Vorrichtung 2 zum Verabreichen von medizinischer Flüssigkeit gemäß der vorliegenden Offenbarung. Insbesondere ist die Vorrichtung 2 als eine Infusionspumpe ausgebildet, beispielsweise als eine Spritzenpumpe oder eine Schlauchpumpe. Die Vorrichtung 2 weist ein Gehäuse 4 sowie zumindest eine, insbesondere bei Gebrauch der Vorrichtung Wärme erzeugende Komponente 6, d.h. eine zu kühlende Komponente, auf. Die Komponente 6 ist innerhalb des Gehäuses 4 angeordnet.

In der dargestellten Ausführungsform weist die Vorrichtung 2 zwei zu kühlende Komponenten 6 auf. Ein Netzteil 8 zur Stromversorgung ist eine Wärme erzeugende Komponente 6 (vgl. Fig. 2). Ein Funkmodul 10 zur Signalübertragung in Form eines WLAN-Moduls ist eine Wärme erzeugende Komponente 6 (vgl. Fig. 2). Alternativ könnte die Komponente 6 auch durch einen Akkumulator zur Stromversorgung, ein anderes Funkmodul wie ein Bluetooth-Modul, ein Antrieb zur Förderung der Flüssigkeit, eine Anzeigeeinheit zur Anzeige betriebsrelevanter Daten und/oder ein Steuergerät zum Steuern der Vorrichtung 2 gebildet sein, auch wenn dies nicht dargestellt ist.

Die Vorrichtung 2 weist einen passiven Kühlkörper 12 auf. Der Kühlkörper 12 ist auf einer Außenseite des Gehäuses 4 angebracht/angeordnet. Der Kühlkörper 12 ist thermisch, insbesondere wärmeleitend, mit der Komponente 6 (bzw. den Komponenten 6) verbunden. Somit wird die Wärme durch Wärmeleitung aus dem Inneren des Gehäuses 4 zu dem Kühlkörper 12 auf der Außenseite transportiert, um dort an die Umgebung abgegeben zu werden.

Vorzugsweise kann der Kühlkörper 12 aus einem Material ausgebildet sein, dessen Wärmeleitfähigkeit größer als 10 W/mK ist, bevorzugt größer als 50 W/mK, weiter bevorzugt größer als 100 W/mK. Vorzugsweise kann der Kühlkörper aus einem Metall ausgebildet sein, insbesondere aus Aluminium oder Kupfer. Zudem kann der Kühlkörper 12 eine Oberfläche aufweisen, die einen Emissionsgrad von größer als 0,8 hat. Alternativ oder zusätzlich kann die Oberfläche des Kühlkörpers 12 eloxiert sein.

Vorzugsweise kann die Vorrichtung 2 ein innerhalb des Gehäuses 4 angeordnetes Wärmeleitprofil 14 aufweisen. Das Wärmeleitprofil 14 ist beispielsweise als ein Wärmeleitblech oder dergleichen ausgebildet. Das Wärmeleitprofil 14 kann, vorzugsweise direkt, d.h. mit mechanischem Kontakt, mit der Komponente 6 (bzw. den Komponenten 6) thermisch verbunden sein. Das Wärmeleitprofil 14 kann, vorzugsweise direkt, d.h. mit mechanischem Kontakt, mit dem Kühlkörper 12 thermisch verbunden sein. Insbesondere kann das Wärmeleitprofil 14 flächig anliegend mit der Komponente 6 und/oder dem Kühlkörper 12 verbunden sein. Vorzugsweise kann das Wärmeleitprofil aus einem Material ausgebildet sein, dessen Wärmeleitfähigkeit größer als 10 W/mK ist, bevorzugt größer als 50 W/mK ist, weiter bevorzugt größer als 100 W/mK ist. Insbesondere kann das Wärmeleitprofil 14 aus einem Metall ausgebildet sein, beispielsweise aus Aluminium oder Kupfer.

Vorzugsweise kann der Kühlkörper 12 eine Kontaktfläche aufweisen, die an einer Kontaktfläche des Wärmeleitprofils 14 anliegt. Alternativ kann die Kontaktfläche des Kühlkörpers 12 mit der Kontaktfläche des oder Wärmeleitprofils 14 über eine thermische Schicht 16, wie einer Wärmeleitpaste, verbunden sein (vgl. Fig. 2). Die Kontaktfläche des Kühlkörpers 12 und/oder die Kontaktfläche des Wärmeleitprofils 14 sind/ist vorzugsweise plan ausgebildet. Der Kühlkörper kann beispielsweise so angeordnet sein, dass er eine Aussparung 18 in dem Gehäuse 4 durchgreift, um das Wärmeleitprofil 14 zu kontaktieren (vgl. Fig. 2).

Beispielsweise kann das Wärmeleitprofil 14 mehrere Wärme erzeugenden Komponenten 6, in der dargestellten Ausführungsform das Netzteil 8 und das Funkmodul 10, mit dem Kühlkörper 12 thermisch verbinden. Vorzugsweise sind die mehreren Komponenten 6, in der dargestellten Ausführungsform das Netzteil 8 und das Funkmodul 10, benachbart angeordnet.

Zudem kann das Wärmeleitprofil 14 beabstandet zu dem Gehäuse 4 angeordnet sein. Das heißt, dass zwischen einer Innenseite des Gehäuses 4 und dem Wärmeleitprofil 14 ein Spalt 20 ausgebildet ist (vgl. Fig. 2), um eine Wärmeübertragung über das Gehäuse 4 zu vermeiden.

Gemäß einer bevorzugten Ausführungsform kann der Kühlkörper 12 einen Koppelabschnitt 22 zur wärmeleitenden Kopplung mit einem externen Gegenkoppelabschnitt (nicht dargestellt) aufweisen. Somit kann der Kühlkörper 12 - zusätzlich zu der Wärmeabgabe über freie/natürliche Konvektion und Wärmestrahlung - die Wärme durch Wärmeleitung an den Gegenkoppelabschnitt übertragen. Vorzugsweise kann der Koppelabschnitt 22 eine Kontaktfläche 24 aufweisen, die vorgesehen und ausgebildet ist, um eine Oberfläche des Gegenkoppelabschnitts, vorzugsweise direkt, insbesondere flächig anliegend, wärmeleitend zu kontaktieren. Die Kontaktfläche 24 und/oder die Oberfläche sind/ist vorzugsweise plan ausgebildet.

Gemäß einer bevorzugten Ausführungsform kann der Kühlkörper 12 Kühlrippen 26 aufweisen, insbesondere um die Oberfläche des Kühlkörpers 12 zu vergrößern.

Die Kühlrippen 26 sind in Gebrauchslage der Vorrichtung vertikal ausgerichtet.

Mit anderen Worten kann der Kühlkörper 12 sowohl eine bereichsweise zur Wärmekonvektion und/oder Wärmestrahlung optimierte Oberflächenstruktur, in Form der Kühlrippen 26, als auch eine bereichsweise zur Wärmeleitung optimierte Oberflächenstruktur, in Form der planen Kontaktfläche 24, aufweisen. Vorzugsweise kann der Koppelabschnitt 22 zwischen den Kühlrippen 26 angeordnet sein.

Die Vorrichtung 2 kann einen Befestigungsabschnitt 28 zur Anbringung der Vorrichtung 2 an einer externen Haltevorrichtung (nicht dargestellt) aufweisen. Beispielsweise kann die Haltevorrichtung als ein Stativ bzw. eine Stativklemme eines Stativs ausgebildet sein. Vorzugsweise kann der Befestigungsabschnitt 28 durch den Koppelabschnitt 22 des Kühlkörpers 12 gebildet sein. Mit anderen Worten fungiert der Koppelabschnitt 22 gleichzeitig zur Befestigung der Vorrichtung 2. Somit kann die Haltevorrichtung als Gegenkoppelabschnitt dienen. So kann der Kontakt zwischen dem Befestigungsabschnitt 28 und Haltevorrichtung als wärmeleitender Kontakt zum Übertragen der Wärme genutzt werden. Beispielsweise kann der Befestigungsabschnitt 28 eine Schiene 30 aufweisen, die vorgesehen und ausgebildet ist, um die Vorrichtung 2, insbesondere werkzeuglos, formschlüssig an der Haltevorrichtung zu befestigen. Insbesondere kann eine durch die Schiene 30 geführte Einsteckrichtung des Befestigungsabschnitts 28 an der Haltevorrichtung vorzugsweise einer Ausrichtung der Kühlrippen 26 und/oder einer Vertikalrichtung entsprechen.

Ferner können am Koppelabschnitt 22 des Kühlkörpers 12 und am Gegenkoppelabschnitt der Haltevorrichtung komplementäre Rastelemente vorgesehen sein, um ein ungewolltes Lösen der Vorrichtung 2 von der Haltevorrichtung zu verhindern.

Gemäß der bevorzugten Ausführungsform kann der Kühlkörper 12, insbesondere der Koppelabschnitt 22, an einer (in Gebrauchslage der Vorrichtung gesehenen) Rückseite des Gehäuses 4 angeordnet sein. Vorzugsweise kann der Kühlkörper 12 einen den Koppelabschnitt 22 abdeckenden und nach außen isolierenden Berührungsschutz (nicht dargestellt) aufweist. Der Berührungsschutz kann einen Einschiebeschlitz für die Haltevorrichtung aufweisen, so dass die Befestigung an der Haltevorrichtung sichergestellt bleibt.

Vorzugsweise kann das Gehäuse 4 im Wesentlichen flüssigkeitsdicht sein, insbesondere lüftungsöffnungsfrei ausgebildet sein. Das heißt, dass das Gehäuse 4 keine Lüftungsschlitze, Lüftungsöffnungen oder dergleichen aufweist, durch die Flüssigkeiten von außen in das Innere des Gehäuses 4 eindringen können.

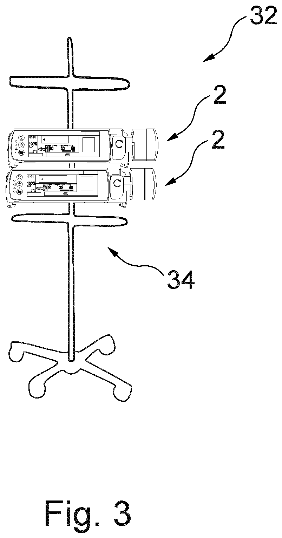

Die Offenbarung betrifft auch ein System 32 aus einer Haltevorrichtung 34, wie einem Stativ und der beschriebenen Vorrichtung 2. Das System 32 ist schematisch in Fig. 3 dargestellt. Die Haltevorrichtung 34 dient demnach als die oben beschriebene externe Haltevorrichtung bzw. als der Gegenkoppelabschnitt. Dabei ist der Kühlkörper 12 der Vorrichtung 2 wärmeleitend mit der Haltevorrichtung 34 verbindbar oder verbunden. Dadurch dienen der Kühlkörper 12 und die Haltevorrichtung 34 als Radiator der Vorrichtung 2, insbesondere der zumindest bei Gebrauch der Vorrichtung 2 Wärme erzeugenden Komponente 6. Vorzugsweise kann die Haltevorrichtung 34 einen Halteradapter aufweisen, der den Kühlkörper 12 und die Haltervorrichtung 34 wärmeleitend verbindet. Insbesondere kann der Halteradapter in den Kühlkörper 12, etwa über die Schiene 30, eingeschoben sein. So wird eine formschlüssige Anbringung der Vorrichtung 2 an der Haltevorrichtung 34 ermöglicht.

Vorzugsweise kann die Haltevorrichtung 34 aus einem Material ausgebildet sein, dessen Wärmeleitfähigkeit größer als 10 W/mK ist, bevorzugt größer als 50 W/mK, weiter bevorzugt größer als 100 W/mK. Vorzugsweise kann die Haltevorrichtung 34 aus einem Metall ausgebildet sein, insbesondere aus Aluminium oder Kupfer.

## Patentansprüche

1. Vorrichtung (2) zur Verabreichung von medizinischer Flüssigkeit, insbesondere Infusionspumpe, mit einem Gehäuse (4), einer zumindest bei Gebrauch der Vorrichtung (2) Wärme erzeugenden Komponente (6), die innerhalb des Gehäuses (4) angeordnet ist, und einem auf einer Außenseite des Gehäuses (4) angebrachten, passiven Kühlkörper (12), der thermisch, insbesondere wärmeleitend, mit der Komponente (6) verbunden ist, wobei der Kühlkörper (12) Kühlrippen (26) aufweist, der Kühlkörper (12) einen Koppelabschnitt (22), der als Teil des Kühlkörpers (12) ausgebildet ist, zur wärmeleitenden Kopplung mit einem externen Gegenkoppelabschnitt aufweist, und der Koppelabschnitt (22) eine, vorzugsweise plane, Kontaktfläche (24) aufweist, die vorgesehen und ausgebildet ist, um eine Oberfläche des Gegenkoppelabschnitts, vorzugsweise direkt, wärmeleitend zu kontaktieren, **dadurch gekennzeichnet, dass**
der Koppelabschnitt (22) zwischen den Kühlrippen (26) angeordnet ist, und die Kühlrippen (26) in Gebrauchslage der Vorrichtung (2) vertikal ausgerichtet sind.

2. Vorrichtung (2) nach Anspruch 1, **gekennzeichnet durch** ein innerhalb des Gehäuses (4) angeordnetes Wärmeleitprofil (14), das, vorzugsweise direkt, mit der Komponente (6) und, vorzugsweise direkt, mit dem Kühlkörper (12) thermisch verbunden ist, wobei der Kühlkörper (12) eine Aussparung in dem Gehäuse (4) durchgreifend angeordnet ist, und das Wärmeleitprofil (14) beabstandet zu dem Gehäuse (4) angeordnet ist.

3. Vorrichtung (2) nach Anspruch 2, **dadurch gekennzeichnet, dass** das Wärmeleitprofil (14) ausschließlich den Kühlkörper (12) und die Komponente (6) kontaktiert.

4. Vorrichtung (2) nach einem der Ansprüche 1 bis 3, **dadurch gekennzeichnet, dass** die Vorrichtung (2) einen Befestigungsabschnitt (28) zur Anbringung der Vorrichtung (2) an einer externen Haltevorrichtung (34), wie an einer Stativklemme eines Stativs, aufweist, wobei der Befestigungsabschnitt (28) durch den Koppelabschnitt (22) des Kühlkörpers (12) gebildet ist und der externe Gegenkoppelabschnitt die Haltevorrichtung (34) ist.

5. Vorrichtung (2) nach einem der Ansprüche 1 bis 4, **dadurch gekennzeichnet, dass** der Befestigungsabschnitt (28) eine Schiene (30) aufweist, die vorgesehen und ausgebildet ist, um die Vorrichtung (2), insbesondere werkzeuglos, formschlüssig an der Haltevorrichtung (34) zu befestigen.

6. Vorrichtung (2) nach Anspruch 5, **dadurch gekennzeichnet, dass** eine durch die Schiene (30) geführte Einsteckrichtung des Befestigungsabschnitts (28) an der Haltevorrichtung (34) vorzugsweise einer Ausrichtung der Kühlrippen (26) und/oder einer Vertikalrichtung der Vorrichtung (2) entspricht.

7. Vorrichtung (2) nach einem der Ansprüche 1 bis 6, **dadurch gekennzeichnet, dass** der Kühlkörper (12), insbesondere der Koppelabschnitt (22), an einer Rückseite des Gehäuses (4) angeordnet ist.

8. Vorrichtung (2) nach einem der Ansprüche 1 bis 7, **dadurch gekennzeichnet, dass** der Kühlkörper (12) einen den Koppelabschnitt (22) abdeckenden und nach außen isolierenden Berührungsschutz aufweist.

9. Vorrichtung (2) nach einem der Ansprüche 1 bis 8, **dadurch gekennzeichnet, dass** das Gehäuse (4) im Wesentlichen flüssigkeitsdicht ist, wobei das Gehäuse (4) vorzugsweise lüftungsöffnungsfrei ausgebildet ist.

10. Vorrichtung (2) nach einem der Ansprüche 1 bis 9, **dadurch gekennzeichnet, dass** der Kühlkörper (12) aus einem Material ausgebildet ist, dessen Wärmeleitfähigkeit größer als 10 W/mK ist, wobei der Kühlkörper vorzugsweise aus einem Metall ausgebildet ist, insbesondere aus Aluminium oder Kupfer, und/oder eine Oberfläche des Kühlkörpers (12) einen Emissionsgrad von größer als 0,8 aufweist und/oder die Oberfläche des Kühlkörpers (12) eloxiert ist.

11. System (32) aus einer Haltevorrichtung (34) und einer Vorrichtung (2) nach einem der Ansprüche 1 bis 10, **dadurch gekennzeichnet, dass** der Kühlkörper (12) der Vorrichtung (2) wärmeleitend mit der Haltevorrichtung (34) verbindbar oder verbunden ist, und der Kühlkörper (12) und die Haltevorrichtung (34) als Radiator der Vorrichtung (2), insbesondere der zumindest bei Gebrauch der Vorrichtung (2) Wärme erzeugenden Komponente (6), dienen.

## Claims

1. A device (2) for administering medical fluid, in particular an infusion pump, comprising a housing (4), a component (6) which is heat-generating at least when the device (2) is in use and which is arranged inside the housing (4), and a passive heat sink (12) which is mounted on an outer side of the housing (4) and which is thermally connected to the component (6), in particular in a heat-conducting manner, wherein the heat sink (12) has cooling ribs (26), the heat sink (12) has a coupling portion (22) formed as a part of the heat sink (12), for coupling in a heat-conducting manner with an external counter-coupling portion, and the coupling portion (22) has a preferably planar contact surface (24) which is provided and configured to contact a surface of the counter-coupling portion, preferably directly, in a heat-conducting manner, **characterized in that**
the coupling portion (22) is disposed between the cooling ribs (26), and the cooling ribs (26) are oriented vertically when the device (2) is in use.

2. The device (2) according to claim 1, **characterized by** a heat-conducting profile (14) arranged within the housing (4), which is thermally connected, preferably directly, to the component (6) and, preferably directly, to the heat sink (12), wherein the heat sink (12) has a recess in the housing (4) that is arranged to penetrate the heat sink (12), and the heat conducting profile (14) is arranged at a distance from the housing (4).

3. The device (2) according to claim 2, **characterized in that** the heat conducting profile (14) exclusively is in contact with the heat sink (12) and the component (6).

4. The device (2) according to any of claims 1 to 3, **characterized in that** the device (2) has a fixing portion (28) for attaching the device (2) to an external holding device (34), such as a stand clamp of a stand, wherein the fixing portion (28) is formed by the coupling portion (22) of the heat sink (12) and the external counter-coupling portion is the holding device (34).

5. The device (2) according to any of claims 1 to 4, **characterized in that** the fixing portion (28) has a rail (30) which is provided and configured to fix the device (2) to the holding device (34), in particular without tools, in a form-fit manner.

6. The device (2) according to claim 5, **characterized in that** an insertion direction of the fixing portion (28) guided by the rail (30) on the holding device (34) preferably corresponds to an orientation of the cooling ribs (26) and/or to a vertical direction of the device (2).

7. The device (2) according to any of claims 1 to 6, **characterized in that** the heat sink (12), in particular the coupling portion (22), is arranged on a rear side of the housing (4).

8. The device (2) according to any of claims 1 to 7, **characterized in that** the heat sink (12) has a protection against contact covering the coupling portion (22) and insulating it from the outside.

9. The device (2) according to any of claims 1 to 8, **characterized in that** the housing (4) is substantially fluid-tight, wherein the housing (4) is preferably configured without ventilation openings.

10. The device (2) according to any of claims 1 to 9, **characterized in that** the heat sink (12) is made from a material having a heat conductivity greater than 10 W/mK, wherein the heat sink is preferably made from a metal, in particular aluminum or copper, and/or a surface of the heat sink (12) has an emissivity of greater than 0.8 and/or the surface of the heat sink (12) is eloxed.

11. A system (32) comprising a holding device (34) and a device (2) according to any of claims 1 to 10, **characterized in that** the heat sink (12) of the device (2) is connectable or is connected to the holding device (34) in a heat-conducting manner, and the heat sink (12) and the holding device (34) serve as a radiator of the device (2), in particular of the component (6), which is heat-generating at least during use of the device (2).

## Revendications

1. Dispositif (2) destiné à l'administration de liquide à usage médical, en particulier une pompe à perfusion, avec un boîtier (4), un composant (6) générant de la chaleur au moins lors d'une utilisation du dispositif (2), lequel composant est disposé à l'intérieur du boîtier (4), et un dissipateur thermique passif (12) installé sur une face externe du boîtier (4), lequel dissipateur thermique est relié thermiquement, en particulier de manière thermiquement conductrice, au composant (6), dans lequel le dissipateur thermique (12) présente des ailettes de refroidissement (26), le dissipateur thermique (12) présente une section de couplage (22) qui est réalisée en tant que partie du dissipateur thermique (12), destinée au couplage thermiquement conducteur à une section de contrecouplage externe et la section de couplage (22) présente une surface de contact (24), de préférence plane, qui est prévue et configurée pour être en contact de manière thermiquement conductrice avec une surface de la section de contrecouplage, de préférence directement, **caractérisé en ce que**
la section de couplage (22) est disposée entre les ailettes de refroidissement (26) et les ailettes de refroidissement (26) sont orientées verticalement dans la position d'utilisation du dispositif (2).

2. Dispositif (2) selon la revendication 1, **caractérisé par** un profilé à conductivité thermique (14) disposé à l'intérieur du boîtier (4), lequel profilé est relié thermiquement, de préférence directement, au composant (6) et, de préférence directement, au dissipateur thermique (12), dans lequel dans le dissipateur thermique (12) présente une encoche disposée de manière traversante dans le boîtier (4) et le profilé à conductivité thermique (14) est disposé à distance du boîtier (4).

3. Dispositif (2) selon la revendication 2, **caractérisé en ce que** le profilé à conductivité thermique (14) est en contact exclusivement avec le dissipateur thermique (12) et le composant (6).

4. Dispositif (2) selon l'une quelconque des revendications 1 à 3, **caractérisé en ce que** le dispositif (2) présente une section de fixation (28) destinée à l'installation du dispositif (2) au niveau d'un dispositif de retenue externe (34), comme au niveau d'une pince pour statif d'un statif, dans lequel la section de fixation (28) est formée par la section de couplage (22) du dissipateur thermique (12) et la section de contrecouplage est le dispositif de retenue (34).

5. Dispositif (2) selon l'une quelconque des revendications 1 à 4, **caractérisé en ce que** la section de fixation (28) présente un rail (30) qui est prévu et configuré pour fixer le dispositif (2), en particulier sans outil, par complémentarité de formes au dispositif de retenue (34).

6. Dispositif (2) selon la revendication 5, **caractérisé en ce qu'**une direction d'insertion, guidée par le rail (30), de la section de fixation (28) au dispositif de retenue(34) correspond de préférence à une orientation des ailettes de refroidissement (26) et/ou à une direction verticale du dispositif (2).

7. Dispositif (2) selon l'une quelconque des revendications 1 à 6, **caractérisé en ce que** le dissipateur thermique (12), en particulier la section de couplage (22), est disposé au niveau d'une face arrière du boîtier (4).

8. Dispositif (2) selon l'une quelconque des revendications 1 à 7, **caractérisé en ce que** le dissipateur thermique (12) présente une protection contre les contacts recouvrant la section de couplage (22) et isolant vers l'extérieur.

9. Dispositif (2) selon l'une quelconque des revendications 1 à 8, **caractérisé en ce que** le boîtier (4) est pour l'essentiel étanche aux fluides, dans lequel le boîtier (4) est de préférence réalisé sans ouvertures d'aération.

10. Dispositif (2) selon l'une quelconque des revendications 1 à 9, **caractérisé en ce que** le dissipateur thermique (12) est réalisé à partir d'un matériau dont la conductivité thermique est supérieure à 10 W/mK, dans lequel le dissipateur thermique est de préférence réalisé en métal, en particulier en aluminium ou en cuivre, et/ou une surface du dissipateur thermique (12) présente un degré d'émission supérieur à 0,8 et/ou la surface du dissipateur thermique (12) est anodisée.

11. Système (32) issu d'un dispositif de retenue (34) et d'un dispositif (2) selon l'une quelconque des revendications 1 à 10, **caractérisé en ce que** le dissipateur thermique (12) du dispositif (2) peut être relié ou est relié de manière thermiquement conductrice au dispositif de retenue (34) et le dissipateur thermique (12) et le dispositif de retenue (34) servent de radiateur du dispositif (2), en particulier du composant (6) générant de la chaleur au moins lors d'une utilisation du dispositif (2).
